# EUROPEAN PATENT SPECIFICATION

(11) **EP 0 759 933 B1**
(45) Date of publication and mention of the grant of the patent: **08.05.2002**
(21) Application number: 96908230.4
(22) Date of filing: 28.03.1996
(51) Int. Cl.: C07K 7/06, C07K 7/08, C07K 14/00

(54) **BETA SHEET FORMING PEPTIDES AND GELS MADE THEREOF**
Peptide, die Beta-Faltblattstrukturen ausbilden und daraus hergestellte Gele
PEPTIDES FORMANT DES ENVELOPPES A PLI BETA ET GELS OBTENUS A PARTIR DE CES PEPTIDES

(30) Priority: 01.04.1995 GB 9506806
(43) Date of publication of application: 05.03.1997
(73) Proprietor: The University of Leeds, Leeds, LS2 9JT (GB); Sofitech N.V., 1180 Brussels (BE)
(72) Inventor: BODEN, Neville, Leeds 17 West Yorkshire LS17 8RX (GB); AGGELI, Amalia, 63 Cardigan Road Leeds LS6 1EB (GB); McLEISH, Thomas, Charles, Buckland, Leeds LS6 2EF (GB)
(74) Representative: Gilholm, Stephen Philip
(86) International application number: GB9600743
(87) International publication number: WO9631528

(56) References cited:
- WO-A-92/09695
- WO-A-95/10535
- POLYM. J. (TOKYO) (1995), 27(8), 819-30 CODEN: POLJB8;ISSN: 0032-3896, 1995, XP000579630 FUKUSHIMA, YASUMASA: "Self-induced helix-sheet conformational transitions of an amphiphilic peptide"
- FEBS LETT. (1991), 291(1), 87-91 CODEN: FEBLAL;ISSN: 0014-5793, 1991, XP002012186 GAY, NICHOLAS J. ET AL: "A leucine-rich repeat peptide derived from the Drosophila Toll receptor forms extended filaments with a. beta.- sheet structure"
- BIOPOLYMERS (1995), 36(3), 391-8 CODEN: BIPMAA;ISSN: 0006-3525, 1995, XP000579636 LEE, SANNAMU ET AL: "Homooligopeptides composed of hydrophobic amino acid residues interact in a specific manner by taking.alpha.-helix or.beta.-structure toward lipid bilayers"

## Description

The invention relates to peptides and polymer tapes derived therefrom for the production of novel materials, particularly, but not exclusively, materials that appear as gels.

It is well known that certain structures are regular features of many proteins, for example, the α-helix or the β-pleated sheet represents such structures.

In the α-helix, polypeptide segments composed of certain amino acids tend to arrange themselves in a regular helical conformation. Thus, the carboxyl oxygen in one peptide bond is hydrogen bonded to the hydrogen on the amino group of the third amino acid away. This results in a helix having 3.6 amino acids per turn and each amino acid residue representing an advance of 0.15nm along the axis of the helix. Each C=O and N-H group in the peptide bonds participates in a hydrogen bond. In this stable arrangement of amino acids the side-chains are positioned along the outside of what is essentially a cylinder.

In the β-pleated sheet, the sheet like structure is created by a series of hydrogen bonds between residues in different polypeptide chains or between residues in different sections of a folded polypeptide. Typically, adjacent polypeptide chains in β-pleated sheets are antiparallel, in other words they run in opposite directions. However, in some structures adjacent chains may run parallel. If a multitude of polypeptide chains participate in the sheet formation the sheet is a rigid wall-like structure. In many proteins, multiple pleated sheets provide the requisite toughness and rigidity.

Febs Lett. (1991), 291(1), 87-91 Coden: Feblal; Issn: 0014-5793, 1991, XP002012186 Gay, Nicholas J et al: 'A leucine-rich repeat peptide derived from the Drosophila Toll receptor forms extended filaments with a beta-sheet structure' describes polypeptide constructs. However, the constructs described by Gay, et al, are fibrils which are substantially rigid with a high persistence length and about 7 to 9nm in width.

WO-A-95 10535 (Scripps Research Inst; Ghadiri Reza M (US)) 20 April 1995 describes cyclic peptides which form molecular tubes. Such polypeptide tubes are distinct from polypeptide β-sheet tapes.

We have discovered that it is possible to exploit the propensity of peptides to self-assemble, via intermolecular hydrogen bonding, into extended β-pleated sheet structures, or β structures. Such structures exist widely as β-sheets or barrels in proteins and also in the "β-plaque" peptide aggregates which are indicators of neurological diseases such as Alzheimer's disease and Bovine Spongiform Encephalopathy. Thus, we have discovered, that it is possible to design oligopeptides, and by this we mean peptides comprising in the order of 4-40 residues which, under appropriate conditions will form long "β-tapes", comprising a β-pleated sheet structure, of a single molecule in thickness. We have surprisingly, discovered that these tapes become engaged or entangled in solution to give fluids viscoelastic properties. This engagement or entanglement gives rise to three-dimensional networks which give rise to materials that appear as gels that are stable at high temperatures. It is of note that these gels, or more specifically gel-like materials, appear as either a chemically cross-link network or they appear to comprise an entangled mesh or web of β-tapes.

We have discovered that the gels have remarkable rheological properties that appear to be dependent on the intimate coupling between molecular self-assembly and dynamics. This means that the gels are susceptible to and responsive to chemical and physical switches which can disrupt the β-tapes and so alter the entanglement properties of the gels resulting in a change in the rheological properties of the gels. Moreover, since the nature of the switching is reversible, this results in the rheological properties of the gels being reversible and so we are able to engineer dynamic systems which respond predictably to preselected switches.

We believe the peptide gels of the invention could find uses in new products and processes in a wide range of industries. The following examples are presented for the purpose of comprehension only and are not intended to limit the scope of the invention.

We believe our gels have application in the oil industry. Specifically, our responsive gels have potential application in both well construction (drilling, completion) and in reservoir stimulation (fracturing, water control). Increased well productivity resulting from reduced impairment of permeability in hydrocarbon-bearing formations would lead to fewer wells needing to be drilled to recover a given amount of oil. This represents significant cost savings when one considers that a typical horizontal well can cost up to £20million. Improvements in reservoir productivity by gel-fluid stimulations can have a dramatic impact on profitability and competitiveness. Current UK north sea oil production is about 1.6m barrels per day. At todays oil prices even a 1% increase in productivity would yield an average revenue of £2000 per day. The cost of effective treatments would therefore be rapidly recovered.

In the pharmaceutical industry there is a need to provide novel drug delivery systems. The gels of our invention having the rheological properties described herein and also the responsiveness, to chemical and physical switches, described herein are of interest to the pharmaceutical industry because they provide novel approaches to drug delivery. In addition, in the pharmaceutical industry, the invention has application in the production of novel wound dressings.

In the personal consumable industry we envisage that the peptide gels of the invention have applications in at least two areas, the first being the structuring of products in a controlled manner and the other being the controlled delivery of functional ingredients to substrates. For example, in the detergent business currently a £108m per year is spent on perfumes for detergent products, however only 1% of the perfume is delivered to the relevant fabric. It therefore follows that the use of triggered delivery through the peptide gels of the invention could potentially allow the delivery of 10% or more of perfume employed giving financial savings of greater than £150m. Similar advantages can be gained in the personal care business where one could arrange for the more efficient delivery of, for example, anti-microbials in the dental market. It will be apparent to those skilled in the art that the invention has relevance in the more effective delivery of agents in personal consumable.

An attractive feature of biopolymers is that they are inherently biocompatible and ultimately biodegradable, this latter feature having obvious environmental implications. They also lend themselves to recycling. For example, a gel of the invention can, in one instance, be recycled by exposing the gel to a physical or chemical switch which is known to alter the properties of the gel and thus transform the gel into a fluid from which additions or contaminants can be extracted prior to reversing the said switch and thus reforming the gel.

In addition, the invention also has application in the production of novel susfactants. For example, it is possible to engineer the peptides comprising the β-tapes of the invention so that, for example on a first side of the peptide there is provided a substantial number of hydrophilic residues and on a second, opposite side, there is provided a substantial number of hydrophobic residues. Engineering of this sort will provide the peptide or, β-tapes with sidedness. Thus, under certain conditions, the first hydrophilic side will be attracted to hydrophilic substances and the second hydrophobic side will attach to hydrophobic substances. Thus we provide a means for bringing theretogether hydrophobic and hydrophilic substances by using the peptides, or β-tapes, as connectors.

In addition, it is also possible to engineer the peptides, or β-tapes, such that they have endedness. For example, it is possible to engineer the peptides so that the first end is substantially hydrophilic and a second opposite end is substantially hydrophobic. Again, such an engineered peptide can be used for the purpose of bringing together disparate substrates.

It will therefore be apparent that tapes engineered as aforedescribed have application as susfactants and can thus be used to stabilise emulsions and/or colloidal dispersions.

Moreover, peptides, or β-tapes, engineered as aforedescribed also have application in the coating of an item or object when said item or object is to be exposed to another substance. For example, one can envisage that the invention has application in the coating of a prosthetic which is to be lubricated with a given substance. It will be possible to engineer the peptide, or β-tape, such that a first end, or side, is adapted to attach to the prosthetic and a second, or opposite, end, or side, is engineered to interact with a lubricant. It will be apparent to those skilled in the art that this is just one example of how the invention can be used in connection with objects or substances which are essentially of a disparate nature.

Furthermore, the invention also has application in the targeted delivery of substances suspended in or embodied in the gel of the invention. For example, it is possible to engineer the peptide with a side residue of group that binds specifically to a given target substance. For example, it is possible to engineer a peptide so that it binds selectively with respiratory tissue and so arrange for the targeted delivery of substances such as anti-asthmatics. It will be apparent to those skilled in the art that the selective engineering of a side residue or group or side group will provide for targeted delivery in a wide range of systems.

It will also be apparent that the selective engineering can involve the provision of a natural or unnatural side residue or side group. For example, a natural side group would comprise a naturally occurring substance such as an amino acid, in contrast an unnatural side group would comprise a substance such as amino Iso butyric acid.

It is therefore an object of the invention to provide a novel material essentially made of peptides dissolved in solution/solvent which can be made to form a gel like structure which, in some, but not all, preferred embodiments, is remarkably insensitive to temperature and which exhibits favourable rheological properties as herein described.

It is a further object of the invention to provide a material which is responsive to chemical and/or physical switches or triggers in order to switch the nature of the material from any one or more of the following phases: a fluid phase to a gel phase to a stiff gel phase and, ideally, vica versa.

It is yet a further object of the invention to provide a material having self-assembling properties so that the material can not only self-assemble but also heal defects which may be created, generated or provided therein.

It is yet a further object of the invention to provide a material which is recyclable and which is also ideally biocompatible and biodegradable.

It is a yet further object of the invention to provide a material which can be selectively engineered so as to interact with dispirit substances and/or to effect targeted delivery or targeted interaction.

According to a first aspect of the invention there is therefore provided a material comprising a plurality of self-assembled peptides characterised in that the self-assembled peptides are in the form of a β-sheet tape structure.

In a preferred embodiment of the invention each of said tapes is between 3-11 nm wide and more preferably 8 nm wide.

Preferably each tape is the approximate thickness of a peptide molecule and thus a single molecule in thickness.

The self-assembled β-sheet tapes may be in an engaged or entangled state to form a gel. The gel may ;be in a solvent of intermediate polarity, e.g. an aqueous medium.

In certain embodiments of the invention, but not all, the material, especially when in the gel state, is remarkably insensitive to temperature and can ideally withstand temperatures up to 85°C or preferably up to 95°C or more preferably up to 250°C; and also down to at least -15°C.

The tolerance to temperature can last indefinitely indicating that the β-tapes are thermally very stable.

In yet a further preferred embodiment of the invention said material comprises either hydrophobic or hydrophilic peptides or a mixture thereof and more preferably comprises an 4-40 residue peptide ideally a 27 residue or 24 residue peptide or a 21 residue peptide.

In an embodiment of the invention said peptides are designed having regard to the following information.

Favourable intermolecular interactions between peptides are essential for formation of self-assembled β-sheet polymers. For example, intermolecular interactions between side-chains with hydrophobic groups - such as isoleucine, leucine or valine, of adjacent β-strand peptides, can effectively stabilise a β-sheet structure. Thus, a peptide which contains numerous hydrophobic side-chains is highly likely to form stable β-sheets in aqueous or non-aqueous polar solvents - such as ethanol. Examples of such peptides are K24, K27 and K27b, whose primary structures are shown in Table 1. These peptides are modelled on the sequences of residues 42-68 of the rat kidney Isk potassium ion-channel. They include the highly hydrophobic transmembrane segment of Isk (residues 45-67).

Specific interactions between pairs of residues, such as between aromatic side-chains - such as tyrosines or phenylalanines, or side-chains with opposite changes - such as glutamic acid and arginine -, which are next to each other on adjacent β-strand peptides, are particularly efficient in establishing a β-sheet structure. Furthermore, their incorporation in a primary peptide structure, provides molecular recognition sites. This is particularly important in the design of β-tapes, where one-dimensional growth of the polymer is required, as opposed to random association of peptide strands which are out of register with respect to each other. The latter situation can result in two dimensional growth of the polymer, and macroscopic structure of quite different morphology from the tapes.

Compatibility of the β-sheet surface and the solvent are also essential for gelation as opposed to peptide precipitation out of solution. For example, K24 forms β-tapes with both polar and apolar groups on their surfaces. Thus, they can form gels in solvents with intermediate polarity, corresponding to values of relative dielectric constants, ∈γ = ∈r=24-62. In solvents with higher or lower polarity than the above values, K24 still forms β-sheet structures which are poorly soluble and can thus come out of solution as precipitate, and gel is not formed.

Typically, in order to obtain an aqueous gel, a peptide should be designed which is more polar than, for example K24. The β-tapes should be adequately soluble in the highly polar aqueous environment, known to favour gelation rather than precipitation.

An approach for empirical design of aqueous gel-forming peptides, involves modelling peptides on water-exposed β-sheet domains of proteins. For example, Lysβ-21 peptide is modelled on the water-soluble β-domain of the proteins leu lysozyme. Lysβ-21 is a peptide which comprises predominantly polar residues. This gives rise to β-sheets with polar residues. This gives rise to β-sheets with polar surfaces, which can easily interact with water and form aqueous gels.

In addition, peptides can be designed to give rise to β-tapes which have distinctive sides. For example, peptides with alternating sequences of polar (e.g. ornithine) - apolar (e.g. valine) side-chains, can form β-tapes with one polar and one apolar side. Because these tapes are partly polar, they can still form aqueous gels, for example the aqueous gel formed by peptide (Drosophila Toll) A2 (Table 1).

Selection of particular non-natural or natural side-chains in the peptide primary structure allows us to control and build responsiveness of the self-assembly process as well as of the properties of the material to external physical or chemical triggers. For example, incorporation of charged side-chains, and of glutamic or anguine can allow us to tune the stability of the β-sheet with ph changes. An example of such a peptide is Lysβ-21.

It is preferred to use a peptide sequence, either naturally occurring or synthetically manufactured, which mimics a sequence of a known peptide such as, for example, a sequence from a known protein such as a transmembrane segment of a putative slow voltage-gated IsK K⁺ channel protein. Typically a peptide comprising the following 27 amino acid residues (KLEALYILMVLGEPGFRLGIMLSYIR), or a 24 residue variants thereof (KLEALYVLGFFGFFTLGIMLSYIR), under certain conditions, give rise to elongated β-tapes approximately 8 nm wide. The conditions required in order to provide the tapes comprise exposure of the peptide to an organic solvent, for example, ethanol, methanol or 2-chloroethanol.

The rheology of the material comprising the β-tapes is, in part, determined by the peptide concentration, for example, a solid-like gel was obtained at 2mM (corresponding to 0.005 peptide volume fraction). Surprisingly, the viscosity and gelation behaviour was found to be relatively insensitive to temperatures up to 85°C in 2-chloroethanol. We have reason to believe that this temperature insensitivity is exhibited at higher temperatures up to 250° C but our observations are of course limited by the boiling point of the organic solvent.

Rheological experiments reveal that the elastic modulus tends to be an order of magnitude greater than the viscous modulus. This data being consistent with a solution of cross-linked or entangled flexible polymer tapes.

In addition, the material of the invention also exhibits a large linear range of stress/shear behaviour. However, at very large strains the behaviour of the material is distinctive, that is to say the growth of stress can suddenly be interrupted at a very large strain, for example of 230% strain.

Interaction between pairs of aromatic side-chain or side-chains with opposite changes which are next to each other or adjacent β-strand peptides are particularly efficient in stabilising a β-sheet structure.

K24 has four aromatic side-chains (phenylalanines) in the middle of the peptide chain. As well as side-chains with opposite changes near either end of the peptide chain. Intermolecular interaction between these side-chains are believed to be important to the formation of the stable β-sheets. In fact we have shown that solvents containing aromatic groups can compete with the interactions of aromatic peptide side-chains and can thus cause the fraction of β-sheet structure of K24 to drop by 20-30%.

This indicates a high degree of flexibility that the β-tapes disrupt at high stress levels.

Experiments also indicate that the material is able to self-assemble and more preferably to self-assemble so as to heal any defects in the structures such as, for example, local twist-like defects.

In preferred embodiments of the invention the material is responsive to chemical switches. For example, we have noted that favourable interactions between the β-tapes and the solution or solvent in which the tapes are suspended is important for gel stability. For example, we report that the addition of lithium chloride can cause certain gels to contract and phase separate as a more concentrated gel phase, alternatively, changing the polarity of the solvent, ie making it more polar (water) or non-polar (chloroform) preserves the β-sheet structure but the gel network is destabilised and precipitation of the peptide occurs, alternatively, in strong hydrogen bonding donor solvents (such as hexafluoroisopropanol) the peptide adopts an α-helical conformation and there is no gelation, alternatively, acetyltion at the N-terminus and amination at the C-terminus of the K27 residue peptide described herein results in a preference for the α-helical conformation and gels are not formed. Alternatively the formation of aqueous gel by the 21-residue peptide [SER1] (modelled on the β-domain of leu lysozyme) can be controlled by pH changes, i.e. pH greater or equal to 11 the gel transforms to fluid.

It follows, that by appropriate peptide design, peptides can be produced which will form β-tapes and gels in a variety of solvents, including water for example, peptides derive from the Drosophila Toll receptor protein, from the Alzheimer amyloid peptide, from desmin filaments and from the β-domain of leu lysozyme all form gels in water.

The rheological properties of these solutions will be intimately connected with the length and stability of β-tapes. The potential for controlling the rheological properties by varying the primary structure of the peptide, changing the solvent, by physical switching, or by addition of chemical triggers, together with the high temperature stability are indicative of exciting prospects for applications of these gels.

In preferred embodiments of the invention the material is responsive to physical switches. For example, the material is responsive to agitation or deformation. Thus, exposure to a shear flow switches the gel from a low to a high modulus state, that it to say the gel gets stiffer. This stiffening of the gel is temporary but can last for a period of, for example 10-15 hours.

In a yet further preferred embodiment of the invention said peptides are engineered to comprise a β-tape that has a first side provided with a substantial number of hydrophilic residues and/or on a second, opposite side a substantial number of hydrophobic resides.

Alternatively, or in addition, said peptide is engineered so as to be provided with a first substantially hydrophilic end and/or a second, opposite, substantially hydrophobic end.

Alternatively, or in addition, said peptide is provided with at least one residue, or group, which is adapted so that said peptide is attached selectively to a predetermined substrate.

The invention will now be described by way of example only with reference to the accompanying figures wherein.

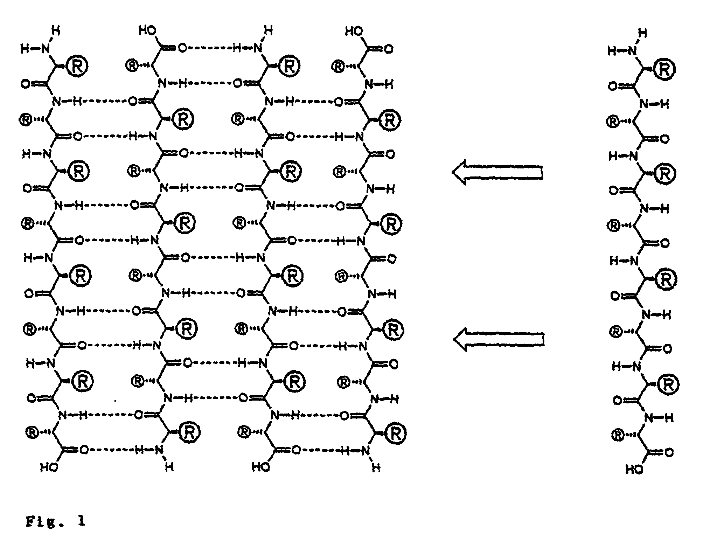

Figure 1 shows the side-by-side assembly of an 8-residue peptide nanotape. The side-chains denoted by big R are arranged above the plane of the sheet and those denoted by little R, below.

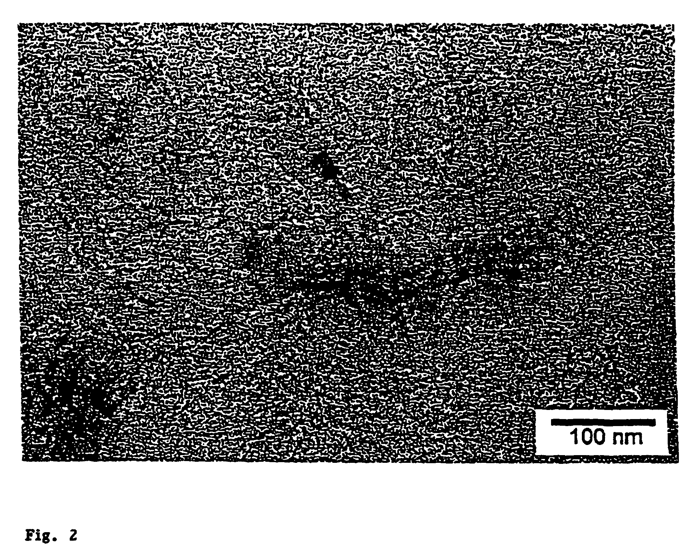

Figure 2 shows a transmission electron micrograph of the peptide gels showing the network of β-tapes or nanotapes. The tapes are approximately 8nm wide and are seen to be engaged or entangled. Very few free ends are apparent, suggesting that the tapes are quite long. The specimens were prepared by addition of the peptide gel (0.5mM in methanol, prepared 24 hours before study to ensure complete formation of the gel and then diluted to 25µM before use) to a 300 mesh size glow-discharged carbon coated copper grid followed by negative staining with uranyl acetate solution (4% w/v in water).

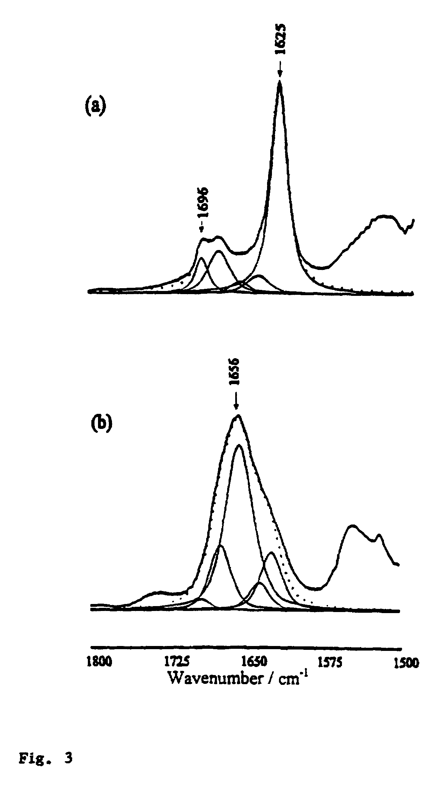

Figure 3 shows band fitted Fourier Transform Infrared Spectra (at 4 cm⁻¹ resolution) of amide I and amide II region of solutions of the 24-residue peptide in (a) methanol solution (1mM), showing the major band at 1625cm⁻¹ and a minor band at 1696cm⁻¹ indicative of peptide molecules assembled as anti-parallel β-strands, (b) hexafluoroisopropanol (1mM), showing the presence of the band at 1656 cm⁻¹ indicative of α-helical conformation. Spectra are averages of 8 scans, recorded at room temperature in a 50 µm CaF₂cell, using a Perkin Elmer 1760X FTIR spectrometer. The spectra shown were obtained by subtraction of the spectrum of the appropriate pure solvent. Components peaks were obtained by second derivative analysis and band-fitting of the absorption spectra.

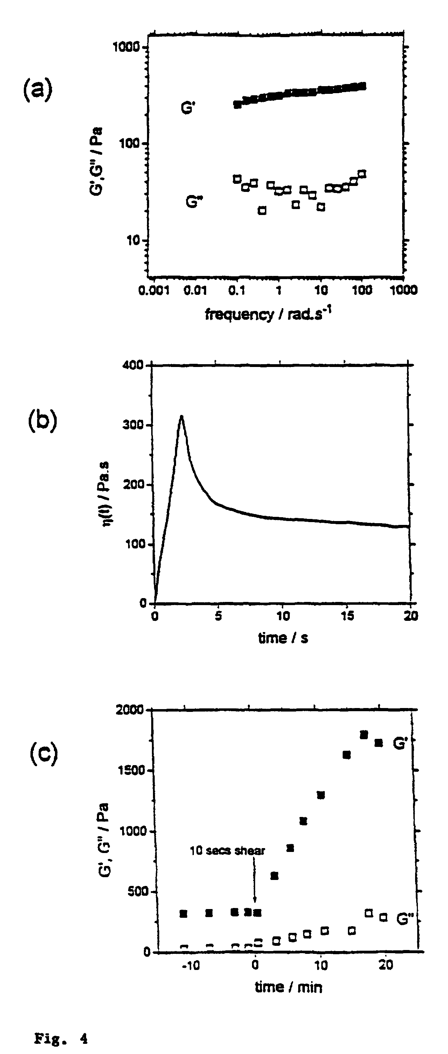

Figure 4 shows mechanical characterisation of the viscoelastic properties of the nanotape gels (24-residue peptide concentration : 10.5 mM in 2-chloroethanol). (a) Typical mechanical spectra (elastic modulus G' and viscous modulus G" versus frequency of applied strain ω) of fully set gels at 24.8°C and 1% strain, which is within the linear viscoelastic region ; (b) stress-strain curve obtained with steady shearing of the sample with shear rate of 1 s⁻¹; (c) dependence of the elastic modulus and the viscous modulus after shearing *for* 10 seconds. A Rheometrics Dynamic Analyser II, with 25mm outer diameter parallel geometry plates has been used for these measurements.

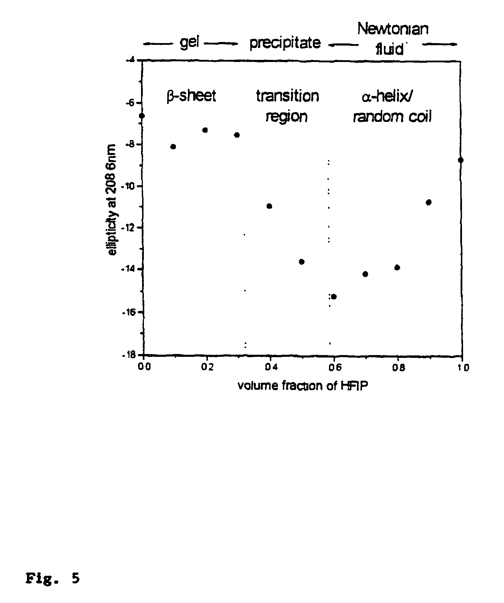

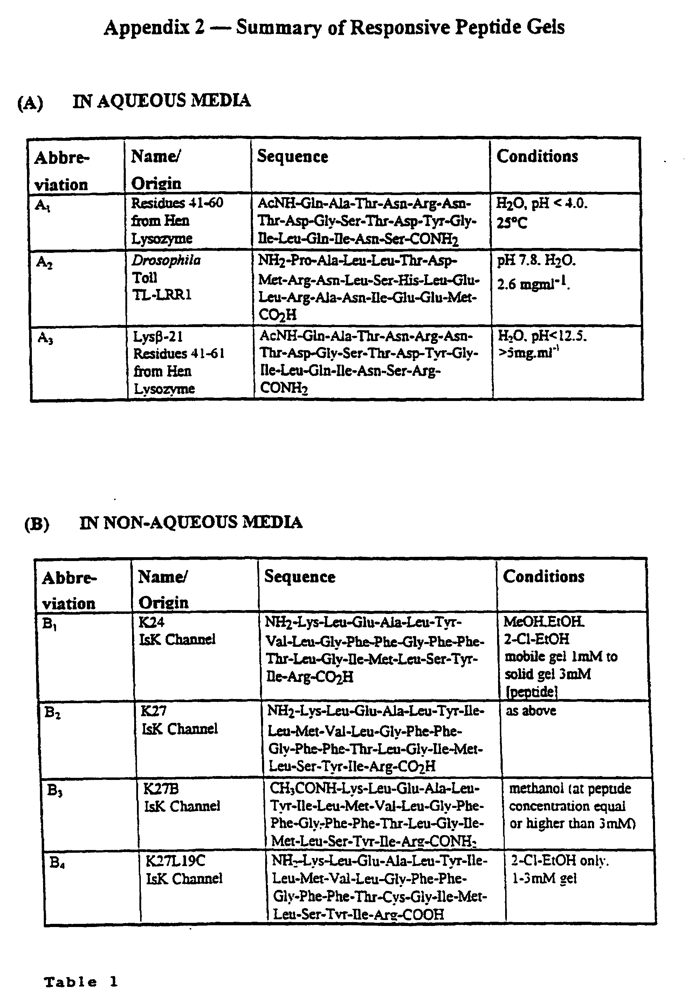

Figure 5 shows β-sheet to α-helix transmission of K24 as a function of a increase in volume action of HFRP in methanol, monitored by CD in 27µM peptides solutions.

Table 1 is a table of responsive peptide gels in (A) aqueous media and (B) non-aqueous media.

Referring to the Figures and firstly to Figure 1, there is shown to the right hand side of the Figure, an 8-residue peptide which, under certain conditions, self-assembles into an 8-residue peptide nanotape or β-tape, shown on the left hand side of the Figure.

In the following experiments we have been working with a 27-residue peptide whose sequence (KLEALYILMVLGFFGFFTLGIMLSYIR) is based on the transmembrane segment of the putative slow voltage-gated IsK K⁺ channel protein. We have also been working on a 24-residue variant of the aforementioned sequence (KLEALYVLGFFGFFTLGIMLSYIR). We have also been working with a 21-residue variant SER-1 as herein described.

In general, gels can be prepared from either hydrophobic peptides (that is they form gels in amphiphilic solvents such as methanol, ethanol and 2-chloroethanol) or hydrophilic peptides (that is they form gels in water) having 8-30-residues.

The hydrophobic peptides have a predominantly hydrophobic segment in the middle of the primary sequence and a few hydrophilic residues on either side of this segment. Peptides B1, B2 and B3 fall into this category. They typically comprise 24-27 residues.

In contrast, hydrophilic peptides typically comprise alternating polar and non-polar residues. Peptide A2 is an example of a hydrophilic gel-forming peptide.

Both the K27-residue (B1) and the K24-residue (B2) peptides were synthesised and purified as follows:

### Materials

Peptide Design. The primary structure of the peptides studied are shown in Figure 1. "K27" is based on the sequence of residues 42-68 of the rat kidney IsK potassium ion channel (Aggeli A., Attwood T., Boden N., Cheng Y., Findlay J.B.C., Hooper I., Kelly M., Knowles P.F., and Turnbull P.J.H., Biochemistry (1994) submitted); Intermolecular interactions between hydrophobic side-chains of adjacent β-strand peptides can stabilise efficiently a β-sheet structure. Thus, a peptide which contains a long hydrophobic sequent in its primary structure is highly likely to form stable sheets. Examples of such peptides are K24 and K27, whose primary structures are shown in Appendix 2. They are modelled on the sequence of residues 42-68 of the rat kidney Isk potassium ion-channel. These peptide sequences include the transmembrane highly hydrophobic segment (residues 45-67) of Isk. Interactions between pairs of aromatic side-chains or side-chains with opposite charges which are next to each other or adjacent β-strand peptides are particularly efficient in stabilising β-sheet structure. K24 has four aromatic side-chains (phenylalanines) in the middle of the peptide chain as well as side-chains with opposite changes near either end of the peptide chain. Intermolecular interaction between these side-chains are believed to be important for the formation of stable β-sheets. In fact we have shown that solvents containing aromatic groups can compete with the interaction of aromatic peptide side-chains and can thus cause the fraction of β-sheet structure of K24 to drop by 20-30%. Compatibility of the β-sheet surface and the solvent are also essential for gelation, as opposed to peptide precipitation out of solution. Thus, K24 forms β-tapes and gels in solvents or mixtures of solvents with intermediate polarity, corresponding to values of rel, dielectric constants, Er: EV=24-62. In solvents with higher or lower polarity than the above K24 still forms β-sheet structures which are poorly soluble and thus come out of solution or precipitate, and gel is not formed.

Typically, in order to obtain aqueous gel, peptide should be designed which is more polar, than for example K24. The β-tapes should be adequately soluble in the highly polar aqueous environment, in order to favour gelation rather than precipitation.

An approach empirical design of aqueous gel-forming peptides involves modelling peptides on water-exposed β-sheet domains of proteins. For example, SER-1 peptide is modelled on the water-soluble β-domain of the protein leu lysozyme.

In addition, peptides with a interaction sequence of polar-apolar side-chains can form β-tapes with one polar and one apolar side. Because these β-tapes are partly polar. They can still form aqueous gel, for example the aqueous gel formed by peptide A2 (Appendix 2).

Incorporation of charged side-chains in the peptide primary structure allows to tune the stability of the β-tape by changes of the pH, and thus control gelation by pH switch. An example of such a peptide is SER-1 (Appendix 2).

### K24 or, K27

Peptide synthesis and purification. Synthesis of peptides employed the continuous-flow, fluorenylmethoxycarbonl (FMOC) polyamide, solid-phase method using the standard manufacturer's protocols on a MilliGen/Biosearch 9050 peptide synthesiser. PepSyn-KA™ resins, pre-derivatised with the FMOC-protected C-terminal amino acid of the desired peptide, and pre-formed FMOC-amino acid-pentafluorophennol (Pfp) esters [or pre-formed dihydroxybenzotriazole esters of FMOC-Ser(tBut) and FMOC-(tBut), where the side-chain hydroxyls are protected as the tertiary butyl derivatives] were used at 4-fold excess to give 0.1 mmole scale synthesis. Couplings were controlled by the counter ion distribution monitoring system (CDM™) to achieve not less then 99% coupling efficiency. Default deprotection protocols were extended to 15 min. after completion of the synthesis the resin was extensively washed with about 150 ml of each of t-amtl alcohol, glacial acetic acid, t-amyl alcohol followed by washing with about 300 ml of diethyl ether. The washed resin was vacuum dried overnight. Resin cleavage and side-chain deprotection was achieved with Reagent K (0.1 ml/mg resin) for 2.5 hours as reported by King et al. (1990) [please see reference Aggeli et al above] expect that 5% water was omitted and 87.5" anhydrous trifluoroacetic acid (TFA) was used instead of 82.5% TFA. The cleaved resin was filtered and washed further with 87.5% TFA. The filtrate was rotary-evaporated at room temperature until all TEA was removed. The gel-like residue was dissolved in hexa-fluoro isopropanol, HFIP, (2 ml), extruded into diethyl ether (50 ml), and centrifuge (microfuge, 4,000 rpm, 5 min, 4°C). The pellet was washed six times, by re-suspension in fresh diethyul ether and centrifugation, before being dried overnight under vacuum. The peptide was dissolved in HFIP (about 1 ml), diluted to 100 ml with de-ionised water and freeze-dried for storage. High resolution ¹H NMR on the peptide dissolved in ²H methanol indicated that it was free of low molecular weight organic impurities. Peptide quantification was carried out using the bichinchoninic acid, BCA (Smith et al 1985 - please see reference Aggeli et al above) or manual ninhydrin (Hirs 1967) assays.

Attempts have been made to purify the peptides by reverse phase HPLC, but difficulties have been encountered due to their highly non-polar character. Initially, a C₁₈ resin was used, but no peptide could be recovered. When a less hydrophobic column (C₄) was used, the peptide was absorbed using 0.1% TFA in methanol as solvent, but low yields of peptide (10%) were achieved during elution, even with the best solvent identified (0.1% TFA in propan-2-ol). Because of these poor yields, HPLC was not used further in the purification. The HPLC elution profile, however, indicated a single peptide species and the sequencing data on the peptide purified by the rigorous extraction and precipitation procedures described was judged acceptable.

### SER1 (93) - Synthesis and Purification

The β-sheet peptide was assembled on an Applied Biosystems 430A automated peptide synthesizer using the base-labile 9-fluorenylmethoxycarbonyl (Fmoc) group for the protection of the α-amino function. Side-chain functional groups were protected by the t-Bu (Asp, Ser, Thr, Tyr), trityl (Asn, Gin), or Pmc (Arg) group. The peptide was assembled on the 4-(2',4'-dimethoxyphenyl-Fmoc-aminomethyl) phenoxy resin to produce a peptide with a C-terminal amide moiety (approximately 0.4 mmol g⁻¹, 0.5 mmol, purchased from Novabiochem) using diisopropylcarbodiimide (DIC)/hydroxybenzotriazole (HOBt) medicated couplings (2 equivalent each of amino acid, DIC, and HObt) which were repeated twice for each residue. Capping of unreacted N-termini was carried out using a 1:1 mixture of acetic anhydride and pyridine in DMF. The Fmoc groups were cleaved by 20% piperidine in DMF, and cycles were modified to allow spectroscopic monitoring of aliquots of each deprotection mixture to allow the progress of synthesis to be monitored. The N-terminus of the peptide was acetylated on the synthesizer using a 4-fold excess of acetic anhydride and pyridine. The peptide-resin was treated with 85% TFA/ 5% H₂O/ 5% ethanedithiol/ 5% thioanisole at room temperature for 1.5h. The resin was then filtered off, and the filtrate was concentrated in vacuo and triturated with ether to afford the fully deprotected peptide amide.

Purification of the peptide was achieved by reverse-phase HPLC on a Dynamax C8 300A preparative column (21.4 x 250 mm, 5-µm particle size) using 0.1% TFA in water as buffer A and 0.1% TFA in acetonitrile as buffer B. A linear gradient between 10% and 30% B in A over 30 min at a flow rate of 20 mL/min was used. The purified peptide was analyzed by reverse-phase HPLC on a Dynamax C8 300A analytical column (4.6 x 250 mm, 5-µm particle size, 1 mL/min flow rate) using a variety of linear gradients between the above mentioned buffers, and it was judged to be >99% pure. The amino acid composition of the peptide was examined by amino acid analysis and was found to be consistent with the expected sequence. A sample of the purified peptide was also analyzed on a VGTOF laser desorption time-of-flight mass spectrometer at a concentration of 22 pmol/µL in α-cyano-4-hydroxycinnamic acid, with an accelerating voltage of 22 kV. The expected molecular mass of the peptide is 2366 Da, and an experimental mass of 2366.1 Da was recorded.

Size-Exclusion Chromatography. The apparent molecular mass of the peptide of the peptide was determined by size-exclusion chromatography at pH 2.5 and 6.8 using TSK 3000SW column. Molecular mass markers used for calibrating the column were aprotinin (6.5kDa), adrencorticotropic hormone (4.5 kDa), insulin A-chain (2.5 kDa), and a 22-residue synthetic peptide of lysozyme (residues 61-82) (2409 Da) which was known to be monomeric and predominantly unstructured under all the conditions studied. Columns were eluted at room temperature with 45 mM sodium phosphate buffer at a flow rate of 0.5 mL/min. Peptides were detected by absorption at 220 and 280 nm.

### K24, K27 and SER-1

Peptide sequence analysis. Peptides were sequenced using both solid-phase and liquid-pulse sequencing strategies. For solid phase sequencing, the peptide (dissolved in 1% HFIP/water) was dried onto a Sequelon™ arylamine membrane disc at 56° C. Covalent coupling was achieved with 1-ethyl-3-(3-dimethylaminopropyl) carbodiimide (10µl, 10 mg/ml) in MES buffer (pH 5.0), dried onto the disc at room temperature (about 30 min). The membrane-coupled peptide was subjected to automated solid-phase Edman-degradation on a MilliGen/Biosearch 6600 Prosequencer. The phenylthiohydantoin-amino acids were identified (at 269) nm by reverse-phase HPLC (Waters SequeTag™ C₈ column) on a Waters 600 system, using a gradient of acetonitrile in 30 mM ammonium acetate, pH 4.8.

Liquid-pulse sequencing was carried out on an Applied Biosystems 477A protein sequencer linked to an Applied Biosystems 120A analyser. TFA-pretreated, glass-fibre discs were pre-conditioned using Biobrene™ (30 µl) which was dried onto the surface of the disc before the disc was subjected to 3 sequencing cycles. Peptide samples (generally 25 µl aliquots of 0.1 - 1 nmol in 1% HFIP/water) were dried onto the pre-conditioned discs under nitrogen. Sequencing was carried out using the approved applied biosystems methodology. Each batch of peptide was subjected to a few cycles of liquid-pulse sequencing to assess the probable purity, using a figure of 90% correct N-terminal residue as the minimum criteria for acceptability of the peptide.

On an industrial scale we expect that the peptides will be manufactured by biological expression systems. For example, it is well known to produce biological peptides such as α-Interferon, a polypeptide of 150 amino acids, by isolation of the relevant gene, incorporation of the gene into a plasmid and expression of the plasmid in a bacterial host under the control of a high efficiency promoter. It is known that expression levels of 25% total cell protein (1gm per litre of culture) have been obtained. The interferon produced in this way is the dominant polypeptide in cytoplasmic inclusion bodies and is also isolated by rupturing the cells and centrifugation. For oligopeptides that is the sequences comprising approximately 20 amino acids expression levels are much lower and in addition the shorter peptides are prone to proteolytic digestion within the cell. For example, expression levels of the 15 residue peptide somatostatin are quoted as 0.05%.

We therefore envisage that high expression levels may be achieved by generating a construct made up of multiple repeats of the relevant peptide or mix of peptides with what we term short linker regions between these sequences, which linker regions may for example be designed to code for methionine or, alternatively, a proteolytic cleavage site so that, in the former instance, once the polypeptide is produced the peptides could be released by cleavage of the expressed polypeptide in acid solution with cyanogen bromide, conditions selective for cleavage C-terminal to methionine, or in the latter instance exposure of the polypeptide to enzyme-mediated digestion.

We also envisage that it may be advantageous to engineer a poly-histidine tail on the polypeptide to enable its single step purification by affinity chromatography.

### β-Tape Structure

In organic solvents such as ethanol, methanol and 2-chloroethanol we have discovered that the aforementioned peptide sequences self-assemble to provide elongated tapes approximately 8 nm wide and a single molecule in thickness. The existence of these β-tapes is revealed directly by transmission electron microscopy as shown in Figure 2. the tapes are seen to be entangled (at distances of approximately 0.1µm) and very few free ends appear to exist, suggesting the tapes are quite long.

The conformation of the peptide chains in the β-tapes is established by their fourier transform infrared spectrum as illustrated in Figure 3a. The prominent band at 1625 cm⁻¹ is identified with the β-strand conformation, whilst the weak band at 1696 cm⁻¹ is a characteristic of β
-strands in the low energy anti-parallel arrangement. Thus it can be seen that the peptides can exist in either a parallel arrangement or an anti-parallel arrangement.

The apparent viscosity as indicated by the flow of the solution in a tube was observed to increase rapidly with peptide concentration, becoming a solid-like gel at 2mM (corresponding to 0.005 peptide volume fraction). Surprisingly, the viscosity and gelation behaviour were found to be relatively insensitive to temperatures up to 85°C in 2-chloroethanol.

### β-Tape Rheology

The rheological properties of the gels were studied and the results are illustrated in Figures 4a and 4b.

It can be seen, that the elastic modulus G' is in order of magnitude larger than the viscous modulus G" (Figure 4A).

The frequency dependence of both is relatively weak. These results are consistent with a solution of engaged or entangled flexible polymer tapes as suggest by the electron micrograph. Another synthetic polymer-like property which contrasts with standard protein gels is the large linear range of the stress-shear behaviour. However, the behaviour at very large strains is distinctive : the growth of stress is suddenly interrupted at a strain of 230% (at 2.3 s in Figure 4b). The linear viscoelastic measurements give directly a value equal to or less than 24 nm for the mesh size of the network. From the value of the limiting strain, a lower limit of 10 nm can be placed on the persistence length (the distance over which the tape randomises its orientation). From these two quantities and the known concentration of the peptide, we can deduce that the tapes are one molecule thick. Assuming a one-dimensional self-assembled tape with an association energy of 30*k*T corresponding to 24 hydrogen bonds, we can estimate an average tape length in excess of a metre. As a consequence, relaxation times by standard mechanisms such as reptation will be unmeasurably long. The gel-like properties therefore stem from the extraordinary lengths of the physically engaged or entangled tapes.

A novel phenomenon was identified following strong shear flow : a subsequently large growth of the gel moduli indicates a switch to a long-lived metasable state. Figure 4c records the growth of both G' and G" to over four times their original values after a 10 seconds shear at a rate of 5s⁻¹. Equilibrium values were restored after a further period of 13 hours. We identify this phenomenon with the creation and healing of local defects in the self-assembling polymer tapes.

The rheological behaviour and the conformation are affected by the primary structure of the peptide as well as the presence of additive and changes of solvent.

### β-Tape Recycling

Figure 5 shows how co-solvents such as HFIP (hexa-fluoro-isopropanol) which is a strong hydrogen-bond donor solvent, can be utilised in order to retrieve peptide in the monomeric state once the β-tape has been formed. This raises opportunities for recycling and reprocessing peptide molecules as well as the reversible control of β-tapes cell assembly.

### Chemical Switches

As mentioned, the gels of the invention are susceptible to chemical triggers which result in changes in the properties of the gels so that they switch from, for example, a first fluid state to a gel state or vica versa.

Addition of salt causes the network to contract and phase separate as a more concentrated gel phase. Changing the polarity of the solvent, ie making it more polar (water) or non-polar ( chloroform), preserves the β-sheet structure, but the gel network is destabilised and precipitation of the peptide occurs. In strong hydrogen-bonding donor solvents (like hexafluoroisopropanol), the peptide adopts an α-helical conformation (Figure 3b) and there is no gelation. Solvents containing aromatic groups cause destabilisation of β-tapes by 20-30% and disruption of the gels. This is believed to be due to competition between intermolecular peptide aromatic side-chain interactions and the aromatic groups of the solvent. Acetylation at the N-terminus and amination at the C-terminus of the 27-residue peptide results in a preference for the α-helical conformation and gels are not formed. It seems that favourable interactions between β-nanotapes and solvent are important for gel stabilisation. By appropriate peptide design, peptides can be produced which will form nanotapes and gels in a variety of solvents, including water. For example, peptides derived from the Drosophila Toll receptor protein, from the Alzheimer amyloid peptide and from desmin filaments and from the β-domain of the leu lysozyme (SER-1).

Chemical switches also include changes in pH, for example, the hydrophilic peptide AcNH-Gln-Ala-Thr-Asn-Arg-Asn-Thr-Asp-Gly-Ser-Thr-Asp-Tyr-Gly-Ile-Leu-Gin-Ile-Asn-Ser-Arg-CONH₂ (SER-1) forms a gel at neutral and acidic pH, whilst it switches to a neutonial fluid at pH higher than 11.

Alternatively, salt can affect the state of gels. For example, the gel of the hydrophobic peptide NH₂-Lys-Leu-Glu-Ala-Leu-Tyr-Val-Leu-Gly-Phe-Phe-Gly-Phe-Phe-Thr-Leu-Gly-Ile-Met-Leu-Ser-Tyr-Ile-Arg-CO₂H (*K24*) contracts upon addition of lithium chloride at a molar ratio of 1 peptide-7 lithium chloride.

Alternatively, surfactant can act as a trigger, so for example the addition of sodium dodecylsulfate (SDS) in NH₂-Lys-Leu-Glu-Ala-Leu-Tyr-Ile-Leu-Met-Val-Leu-Cly-Phe-Phe-Gly-Phe-Phe-Thr-Leu-Gly-Ile-Met-Leu-Ser-Tyr-Ile-Arg-CO₂H (*K27*) peptide gel at a molar ratio: 1 K27-350 SDS, destroys the gel.

Alteratively still solvents such as for example the addition of 50% v water in B1 gel (see Table 1) destroys the gel. Alternatively, the addition of 50% v 1,1,1,3,3,3-hexafluoropropanol-2 in B1 (see Table 1) gel destroys the gel.

### Physical Switches

As also mentioned, the gels of the invention are susceptible to physical triggers which result in changes in the properties of the gels so that they switch from, for example, a first gel-like state to a second stiffer gel-like state. The physical trigger comprises any means of agitation, deformation or shearing of the gel.

The gels of the invention have a moduli which range from 1Pa to a 1000Pa, and can be increased to 2500Pa, and possibly as high as 10,000Pa, though the latter high values are typically only experienced temporarily after shear flow. The gels of the invention tend to be nearly linear in response up to a 200% strain.

The increase in modulus, up to 10 times its ambient value, typically over a time period in the order of 25 minutes, stiffens the gel and the gel will remain stiffened for a prolonged period of time, for example, 10 hours of so. During this period the gels tend to be more strain-dependent.

It can therefore be seen that the gel or gel-like materials of the invention are responsive to both chemical and physical switches, which switches alter the rheological properties of the gels which alteration in properties can be exploited to advantage.

The invention therefore concerns a novel material having selectively modulatable properties.

## Claims

1. A material comprising a plurality of self-assembled peptides **characterised in that** the self-assembled peptides are in the form of a β-sheet tape structure.

2. A material according to claim 1 **characterised in that** the self-assembled β-sheet tape is a single molecule in thickness.

3. A material according to claim 1 or 2 **characterised in that** a plurality of the self-assembled β-sheet tapes are in an engaged or entangled state to form a gel.

4. A material according to claim 3 **characterised in that** the plurality of self-assembled β-sheet tapes are in an engaged or entangled state to form a gel in a solvent of intermediate polarity.

5. A material according to claim 4 **characterised in that** the plurality of self-assembled β-sheet tapes form a gel in an aqueous medium.

6. A material according to any of claims 1 to 5 **characterised in that** the peptides are between 4-40 amino acid residues in length.

7. A material according to any of claims 1 to 6 **characterised in that** a plurality of said peptides comprise amino acid residues having hydrophobic side-chains.

8. A material according to claim 7 **characterised in that** said amino acid residues comprise isoleucine, leucine or valine.

9. A material according to any of claims 1 to 6 **characterised in that** a plurality of said peptides comprise amino acid residues of an aromatic nature.

10. A material according to claim 9 **characterised in that** said amino acid residues are tyrosine or phenylalanine.

11. A material according to any of claims 1 to 6 **characterised in that** a plurality of said peptides comprise amino acid residues of opposite charge.

12. A material according to claim 11 **characterised in that** said amino acid residues are glutamic acid and/or arginine.

13. A material according to any of claims 1 to 6 **characterised in that** a plurality of said peptides have a sequence of amino acid residues with alternate polar and apolar side-chains.

14. A material according to any of claims 1 to 13 **characterised in that** at least some of said peptides comprise the K27 peptide.

15. A material according to any of claims 1 to 13 **characterised in that** at least some of said peptides comprise the K24 peptide.

16. A material according to any of claims 1 to 13 **characterised in that** at least some of said peptides comprise the SER-1 peptide.

17. A material according to any of claims 1 to 16 comprising any one or more of the peptides selected from amino acid residues 41-60 from Hen Lysozome; Drosophila Toll TL-Lrr1; Lys β-21 amino acid residues 41-61 from Hen Lysozome; K24; K27; K27B and K27 L19C.

18. A material according to claims 1 or 3 **characterised in that** its rheological properties can be changed on exposure to physical triggers, selected from agitation, deformation and shearing; and/or chemical triggers selected from pH, strong hydrogen-bond donor solvent, use of salt, changing the polarity of the solvent, use of a solvent containing aromatic groups and surfactants.

19. A material according to claim 4 **characterised in that** the change in rheological properties is reversible.

20. A material according to claims 1 or 3 **characterised in that** the material is recyclable, biocompatible and/or biodegradable.

21. A material according to claims 1 or 3 **characterised in that** the material is suitable for use in the oil industry.

22. A material according to claim 21 **characterised in that** the material is suitable for use in well construction or reservoir stimulation.

23. A material according to claim 22 **characterised in that** the material is suitable for use in well drilling or completion, or reservoir fracturing or water control.

24. A material according to claims 1 or 3 **characterised in that** the material is suitable for use in the controlled delivery of functional ingredients.

25. A material according to claims 24 **characterised in that** the material is used in a drug delivery system.

## Patentansprüche

1. Ein Material bestehend aus einer Vielfalt von selbst- zusammengesetzten Peptiden, charakterisiert dadurch, daß die selbst- zusammengesetzten Peptide in der Form von einer β-Faltblattstruktur sind.

2. Ein Material nach Anspruch 1, charakterisiert dadurch, daß die selbstzusammengesetzte β-Faltblattstruktur ein Molekül dick ist.

3. Ein Material nach Anspruch 1 oder 2, charakterisiert dadurch, daß eine Vielfalt der selbst-zusammengesetzten β-Faltblattstrukturen in einer ineinander greifenden oder ineinander verwickelten Form sind und somit ein Gel bilden.

4. Ein Material nach Anspruch 3, charakerisiert dadurch, daß eine Vielfalt der selbst-zusammengesetzten β-Faltblattstrukturen in einer ineinander greifenden oder ineinander verwickelten Form sind und somit ein Gel in einem Lösungsmittel von mittlerer Polarität bilden.

5. Ein Material nach Anspruch 4, charakterisiert dadurch, daß die Vielfalt der selbst-zusammengesetzten β-Faltblattstrukturen ein Gel in einem wasserhaltigen Medium bilden.

6. Ein Material nach irgendeinen der Ansprüche 1 bis 5, charakterisiert dadurch, daß die Peptide zwischen 4-40 Aminosäurenrückstände in Länge sind.

7. Ein Material nach irgendeinen der Ansprüche 1 bis 6, charakterisiert dadurch, daß die Vielfalt der genannten Peptide aus Aminosäurenrückständen mit hydrophobischen Seitenketten besteht.

8. Ein Material nach Anspruch 7, charakterisiert dadurch, daß die genannten Aminosäurenrückstände aus Isoleucin, Leucin und Valin bestehen.

9. Ein Material nach irgendeinem der Ansprüche 1 bis 6, charakterisiert dadurch, daß die Vielfalt der genannten Peptide aus Aminosäurenrückständen von einer aromatischen Natur bestehen.

10. Ein Material nach Anspruch 9, charakterisiert dadurch, daß die genannten Aminosäurenrückstande Tyrosin oder Phenylalanin sind.

11. Ein Material nach irgendeinen der Ansprüche 1 bis 6, charakterisiert dadurch, daß eine Vielfalt von den genannten Peptiden aus Aminosäuren von entgegengesetzter Ladung bestehen.

12. Ein Material nach Anspruch 11 dadurch charakterisiert, in dem die genannten Aminosäurenrückstände Glutaminsäure und/ oder Arginin sind.

13. Ein Material nach irgendeinen der Ansprüche 1 bis 6 dadurch charakterisiert, in dem die Vielfalt der genannten Peptide eine Sequenz von Aminosäurenrückstände mit abwechselnden polaren und apolaren Seitenketten haben.

14. Ein Material nach irgendeinen der Ansprüche 1 bis 13 dadurch charakterisiert, in dem mindestens einige der genannten Peptide das K27 Peptid beinhalten.

15. Ein Material nach irgendeinen der Ansprüche 1 bis 13 dadurch charakterisiert, in dem mindestens einige der genannten Peptide das K24 Peptid beinhalten.

16. Ein Material nach irgendeinen der Ansprüche 1 bis 13 dadurch charakterisiert, in dem mindestens einige der genannten Peptide das SER-1 Peptid beinhalten.

17. Ein Material nach irgendeinen der Ansprüche 1 bis 16, bestehend aus einem oder mehreren der Peptide, ausgewählt von den Aminosäurenrückständen 41-60 vom Hühner-Lysosom; Drosophila Toll TL-Lrrl; Lys β-21 Aminosäurerückstände 41-61 vom Hühner Lysosom; K24, K27; K27B and K27 L19C.

18. Ein Material nach Ansprüchen 1 oder 3, dadurch charakterisiert, in dem die rheologischen Eigenschaften verändert werden können, wenn sie physikalischen Auslösern ausgesetzt sind, ausgesucht von Agitation, Deformation und Reißen; und/ oder chemikalische Auslöser, ausgesucht von pH, Hauptlösungsmittel mit starker Wasserstoffbindung, Gebrauch von Salz, Veränderung der Polarität vom Lösungsmittel, Gebrauch von einem Lösungsmittel welches aromatische Gruppen enthält und oberflächenaktive Stoffe.

19. Ein Material nach Anspruch 4, dadurch charakterisiert, in dem die Veränderung der rheologischen Eigenschaften umkehrbar ist.

20. Ein Material nach Ansprüchen 1 oder 3, dadurch charakterisiert, in dem das Material wiederverwertbar, biokompatibel und/ oder biodegradierbar ist.

21. Ein Material nach Ansprüchen 1 oder 3, dadurch charakterisiert, in dem das Material in der Ölindustrie benutzbar ist.

22. Ein Material nach Anspruch 21, dadurch charakterisiert, in dem das Material in der Brunnenkonstruktion oder Reservoirstimulation benutztbar ist.

23. Ein Material nach Anspruch 22, dadurch charakterisiert, in dem das Material beim Drillen vom Bohrloch oder Fertigung, oder Reservoirfraktur oder Wasserkontrolle benutzbar ist.

24. Ein Material nach Ansprüchen 1 oder 3, dadurch charakterisiert, in dem das Material für die kontrollierte Lieferung von funktionellen Zutaten benutzbar ist.

25. Ein Material nach Anspruch 24, dadurch charakterisiert, in dem das Material in einem Drogenlieferungssystem angewendet werden kann.

## Revendications

1. Matériau comprenant une pluralité de peptides auto-assemblés, **caractérisé en ce que** les peptides auto-assemblés sont sous la forme d'une structure en bande feuille bêta.

2. Matériau selon la revendication 1, **caractérisé en ce que** la bande feuille bêta auto-assemblée présente une seule molécule en épaisseur.

3. Matériau selon la revendication 1 ou 2, **caractérisé en ce qu'**une pluralité des bandes en feuilles bêta auto-assemblées est dans un état engagé ou enchevêtré pour former un gel.

4. Matériau selon la revendication 3, **caractérisé en ce que** la pluralité des bandes en feuilles bêta auto-assemblées est dans un état engagé ou enchevêtré pour former un gel dans un solvant de polarité intermédiaire.

5. Matériau selon la revendication 1, **caractérisé en ce que** la pluralité des bandes en feuilles bêta auto-assemblées forme un gel en milieu aqueux.

6. Matériau selon l'une des revendications 1 à 5, **caractérisé en ce que** les peptides présentent une longueur entre 4 et 40 résidus acides aminés.

7. Matériau selon l'une des revendications 1 à 6, **caractérisé en ce qu'**une pluralité desdits peptides comporte des résidus acides aminés ayant des chaînes latérales hydrophobiques.

8. Matériau selon la revendication 7, **caractérisé en ce que** lesdits résidus acides aminés comportent isoleucine, leucine ou valine.

9. Matériau selon l'une des revendications 1 à 6, **caractérisé en ce qu'**une pluralité desdits peptides comporte des résidus acides aminés de nature aromatique.

10. Matériau selon la revendication 9, **caractérisé en ce que** lesdits résidus acides aminés sont tyrosine ou phénylalanine.

11. Matériau selon l'une des revendications 1 à 6, **caractérisé en ce qu'**une pluralité desdits peptides comporte des résidus acides aminés de charge opposée.

12. Matériau selon la revendication 11, **caractérisé en ce que** lesdits résidus acides aminés sont de l'acide glutamique et/ou de l'arglnine.

13. Matériau selon la revendication 1, **caractérisé en ce qu'**une pluralité desdits peptides comporte une séquence de résidus acides aminés avec des chaînes latérales alternées polaires et apolaires.

14. Matériau selon l'une des revendications 1 à 13, **caractérisé en ce qu'**au moins un desdits peptides comporte un peptide K27.

15. Matériau selon l'une des revendications 1 à 13, **caractérisé en ce qu'**au moins un desdits peptides comporte un peptide K24.

16. Matériau selon l'une des revendications 1 à 13, **caractérisé en ce qu'**au moins quelques uns desdits peptides comportent le peptide SER-1.

17. Matériau selon l'une des revendications 1 à 16, **caractérisé en ce qu'**il comporte un ou plusieurs des peptides choisis parmi des résidus acides aminés 41-60 de Hen Lysozome ; Drophilia Toll TL-LrLL; des résidus acides aminés Lys béta-21 41-61 de Hen Lysozome ; K24 ; K27 ; K27B et K27 L 19C.

18. Matériau selon l'une des revendications 1 à 3, **caractérisé en ce que** ses propriétés rhéologiques peuvent être changées par exposition à des déclencheurs physiques, choisis parmi l'agitation, la déformation et le déchirement, et/ou des déclencheurs chimiques choisis parmi pH, de solvants à fort lien hydrogène, l'utilisation de sets, le changement de polarité du solvant, l'utilisation de solvant contenant des groupes aromatiques et des surfactants.

19. Matériau selon la revendication 4, **caractérisé en ce que** le changement de propriété rhéologique est réversible.

20. Matériau selon l'une des revendications 1 à 3, **caractérisé en ce que** le matériau est recyclable, biocompatible et/ou biodégradable.

21. Matériau selon l'une des revendications 1 à 3, **caractérisé en ce qu'**il est susceptible d'être utilisé dans l'industrie pétrolière.

22. Matériau selon la revendication 21, **caractérisé en ce qu'**il est susceptible d'être utilisé dans la construction de puits ou dans la stimulation de puits.

23. Matériau selon la revendication 21 **caractérisé en ce qu'**il est adapté à l'utilisation dans le forage ou cimentation de puits, ou la fracturation de puits ou contrôle de l'eau.

24. Matériau selon l'une des revendications 1 à 3, **caractérisé en ce qu'**il est adapté à l'utilisation dans la délivrance contrôlée d'ingrédients fonctionnels.

25. Matériau selon la revendication 24, **caractérisé en ce qu'**il est utilisé dans un système de distribution de médicament.
